(19) Europäisches Patentamt
European Patent Office
Office européen des brevets

(11) Publication number: 0 002 849
B1

# (12) EUROPEAN PATENT SPECIFICATION

(45) Date of publication of patent specification: 27.01.82

(21) Application number: 78200350.3

(22) Date of filing: 06.12.78

(51) Int. Cl.³: C 07 C 21/04, C 07 C 17/26, C 07 C 69/743 //C07F9/22

## (54) Process for the preparation of di-(halo)-vinyl compounds and di-(halo)-vinyl compounds produced thereby.

(30) Priority: 16.12.77 GB 5246777

(43) Date of publication of application: 11.07.79 Bulletin 79/14

(45) Publication of the grant of the European patent: 27.01.82 Bulletin 82/4

(84) Designated Contracting States: BE CH DE FR GB IT NL

(56) References cited:
GB - A - 1 410 165
GB - B - 1 413 491

Chemical Abstracts vol. 75, no. 13 1971 Abstract no. 88 029y, G. LAVIELLE et al

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Verbrugge, Pieter Adriaan
Badhuisweg 3
NL-1031 CM Amsterdam (NL)
Inventor: Kramer, Petrus Anthonius
Badhuisweg 3
NL-1031 CM Amsterdam (NL)

(74) Representative: Keuzenkamp, Abraham et al,
P.O. Box 302
NL-2501 CH Den Haag (NL)

EP 0 002 849 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of di-(halo)-vinyl compounds and di-(halo)-vinyl compounds produced thereby

This invention relates to a process for the preparation of di-(halo)-vinyl compounds. Di-(halo)-vinylcyclopropyl compounds are particularly useful as intermediates in the manufacture of insecticidally active compounds known as synthetic pyrethroids. As these "pyrethroids" combine exceptionally good insecticidal properties with a very low mammalian toxicity, they are of considerable interest to the agrochemical industry and much effort has been expanded in finding economic routes to them and to their intermediates.

British patent specification 1,413,491 discloses the preparation of dihalovinylcyclopropyl compounds by reacting 3-formyl-2,2-dimethylcyclopropane carboxylate with a dihalomethylenephosphorane (which can be prepared by reaction of a triorganophosphine, normally triphenylphosphine, with a carbon tetrahalide). The specification discloses an essentially one-step process giving rise to relatively low yields of dihalovinylcyclopropyl compounds (see Examples 15 and 16).

Lavielle *et al* in Bull. Soc. Chim. Fr., No. 6, 1971, Pages 2047 to 2053 describe the single step synthesis of dichlorovinyl compounds by reacting together tri(dimethylamino)phosphine, carbon tetrachloride and an aliphatic aldehyde in tetrahydrofuran at a temperature of −78°C. Yields in the range of 50 to 55% are obtained.

It has now been found that di-(halo)-vinyl compounds can be obtained in high yield via a two-step process involving the use of a tri(dialkylamino)phosphine at relatively accessible temperatures.

Accordingly, the present invention provides a process for the preparation of a compound of the general formula:

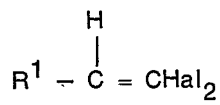

(I)

wherein Hal represents a halogen atom and $R^1$ and alkyl group or a substituted cyclopropyl group of the general formula:

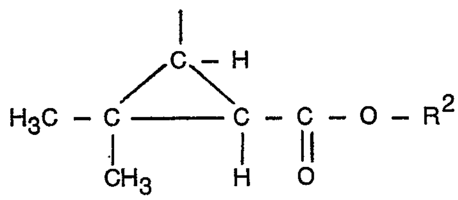

(II)

wherein $R^2$ represents a substituted or unsubstituted alkyl group, which process comprises reacting a tri(dialkylamino)phosphine with a tetrahalomethane and an aldehyde of the general formula

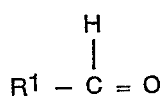

(III)

wherein $R^1$ has the same meaning as in the general formula I, in the presence of a substantially inert solvent, characterised in that (a) as a first step the tri(dialkylamino)phosphine is reacted with the tetrahalomethane in the absence of the aldehyde and (b) the product resulting from the first step is reacted with the aldehyde, steps (a) and (b) both being effected at temperatures in the range from −50°C to +50°C.

Preferably steps (a) and (b) are both effected at temperatures in the range from −20°C to +35°C. The alkyl groups present in the tri-(dialkylamino)phosphine may be the same or different and linear or branched. The alkyl groups are suitably the same, have preferably less than six carbon atoms and more preferably less than three. Tri-(diethylamino)phosphine and tri-(dimethylamino)phosphine are most preferred.

Tri(dialkylamino)phosphines may be prepared by reaction of a dialkylamine with a phosphorous trihalide, as described in "Organic Synthesis", Coll. Vol. V (1973), 602—603. This reaction results in the formation of a solution of the tri(dialkylamino)phosphine which also contains precipitated dialkylammonium halide. According to a feature of the present invention a tri(dialkylamino)phosphine may be prepared by reacting a dialkylamine with a phosphorous trihalide in the presence of a substantially inert solvent. The resulting reaction mixture can then be washed with water to remove

unwanted by-products (whether or not after prior separation of the precipitated dialkylammonium halide) and the tri(dialkylamino)phosphine dissolved in the washed solution reacted with the halomethane. It is not necessary to separate the precipitated dialkylammonium halide prior to washing, because this salt is water-soluble. The yield of the compound of formula (I) can be further enhanced by drying the washed liquid, for example, over a solid drying agent, such as anhydrous sodium sulphate or anhydrous magnesium sulphate.

Another attractive feature of the process according to the present invention is that it may be carried out in the presence of an alkane solvent, for example, alkane solvents with a boiling point or boiling range up to 200°C. This also applies to the said reaction between a dialkylamine and a phosphorous trihalide. Examples of alkane solvents are pentane, hexane, heptane, octane and nonane. Mixture of alkanes are very suitable, for example, gasolines having a boiling range from 62°C to 82°C or from 80°C to 110°C. If desired, the process may be carried out in substantially inert solvents other than alkanes, for example, in tetrahydrofuran.

Examples of tetrahalomethanes are carbon tetrachloride, carbon tetrabromide, carbon tetra-iodide, bromotrichloromethane (forming dichlorocarbene) and dibromodifluoromethane (forming difluorocarbene). Very good results have been obtained with carbon tetrachloride. These halomethanes may also act as solvent or co-solvent for the process according to the invention.

$R^1$ in formulae (I) and (III) preferably represents an alkyl group containing less than 25 carbon atoms, more preferably less than 15 carbon atoms, or a substituted cyclopropyl group of formula II, in which $R^2$ is an alkyl group containing less than 6, especially less than 3, carbon atoms. The preferred aldehyde reactants are *n*-hexanal, *n*-heptanal, *h*-decanal and ethyl 3-formyl-2,2-dimethylcyclopropane carboxylate. The cyclopropylaldehydes may have a *cis*- or *trans*-structure or a mixture of such stereoisomers, or may be an optical isomer or mixture of optical isomers.

If desired, the alkyl group represented by $R^2$ in formula II may be a methyl group carrying a substituent which produces pyrethroid insecticidal activity of the type disclosed in British patent specification 1,413,491, for example, a 3-phenoxy-phenyl group, a cyano group, or preferably both such groups.

The process may be carried out by adding a tri(dialkylamino)phosphine to a halomethane, in a solvent that is substantially inert, for example, in an alkane solvent and stirring the mixture thus obtained until the first step has been substantially completed, which may take from 1 to 60 minutes. Then, the aldehyde of formula III is added to the mixture and stirring continued for 1 to 60 minutes until the second step has been completed. Dihalophosphoranes and phosphine oxides can be removed from the reaction mixture by washing. This washing can be carried out with water when tri(dimethylamino)phosphine has been used, but when a tri(dialkylamino)phosphine having two or more carbon atoms in the alkyl groups has been used, dilute aqueous hydrochloric acid is more suitable than water. Therefore, tri(dimethylamino)phosphine is the most preferred tri(dialkylamino)phosphine. The washed reaction mixture is dried and the solvent is evaporated from the dried solution to leave a residue, which may be further purified, for example by distillation, to obtain the compound of formula I in a pure state.

The process is of particular interest when the aldehyde of formula III is an alkyl (1R, cis)-3-formyl-2,2-dimethylcyclopropane carboxylate and the compound generating the dihalocarbene is carbon tetrachloride, because the ethyl 2-(2,2-dichlorovinyl)-3,3-dimethylcyclopropane carboxylate then formed is a precursor of "pyrethroids", for example, of *alpha*-cyano-3-phenoxybenzyl 2-(2,2-dichlorovinyl)-3,3-dimethylcyclopropane carboxylate, a highly active insecticidal compound.

The Examples further illustrate the present invention. The experiments described below were carried out in a three-necked round-bottomed flask provided with a dropping funnel, magnetic stirrer, thermometer, reflux condenser, calcium chloride tube and an inlet for nitrogen. A cooling bath was present to provide the necessary cooling. Yields and purities were determined by means of gas-liquid chromatography and nuclear magnetic resonance (NMR) spectroscopy.

## Example I

*Preparation of 1,1-dichloro-1-octene in pentane*

Tri(dimethylamino)phosphine (22 mmol.) was added with stirring at a temperature of −25°C to a solution of carbon tetrachloride (20 mmol.) in pentane (25 ml). Subsequent removal of the cooling bath allowed the temperature to rise to −10°C (10 minutes), followed by a sudden temperature increase to −5°C with simultaneous formation of a white precipitate.

This indicated the end of the first step. At −5°C n-heptanal (10 mmol.) was added dropwise to the suspension over a period of three minutes, which resulted in disappearance of part of the precipitate and in a temperature rise to +5°C. The mixture was stirred for a further 5 minutes at 5°C and then allowed to adopt a temperature of 15°C, which took 15 minutes. This ended the second step. The reaction mixture contained the title compound in a yield of 91% at a conversion of n-heptanal of 97%. The reaction mixture was washed with water (25 ml), the washed liquid was dried over anhydrous sodium sulphate, the solvent was evaporated from the dried liquid and the residue obtained was found to contain the title compound in a yield of 71%. The content of the title compound in the residue was 92%.

Comparative Experiment A

A solution of tri(dimethylamino)phosphine (22 mmol.) in pentane (10 ml) was added with stirring over a period of 5 minutes at a temperature of −20°C to a solution of heptanal (10 mmol.) and carbon tetrachloride (20 mmol.) in pentane (15 ml). The mixture obtained was allowed to adopt a temperature of +15°C, which lasted 15 minutes — the conversion of n-heptanal was then 80% — and washed with water (25 ml). The washed mixture was dried over anhydrous sodium sulphate, the solvent was evaporated and the residue obtained was found to contain the title compound of Example I in a yield of 10%.

Comparison with Example I shows that the two-step procedure of Example I affords the title compound of this example in a considerably higher yield.

Example II

*Preparation of 1,1-dichloro-1-octene in tetrahydrofuran*

Tri(dimethylamino)phosphine (22 mmol.) was added dropwise over a period of 5 min. with stirring at a temperature of −10°C to a solution of carbon tetrachloride (20 mmol.) in tetrahydrofuran (25 ml). The temperature was not allowed to exceed +5°C. A precipitate formed. This ended the first step. The temperature was lowered to −10°C, n-heptanal (10 mmol.) was added and the temperature of the mixture was allowed to rise to +5°C, which lasted five minutes. This ended the second step; the conversion of n-heptanal was 98%. The reaction mixture was mixed with water (25 ml) and the mixture thus formed was extracted with three 25 ml portions of pentane. The solvent was evaporated from the combined extract phases to give a residue containing the title compound in a yield of 82%. The content of the title compound in the residue was 91%.

Comparative Experiment B

Tri(dimethylamino)phosphine (22 mmol.) was added with stirring over a period of three minutes to a solution of n-heptanal (10 mmol.) and carbon tetrachloride (20 mmol.) in tetrahydrofuran (25 ml), at a temperature of −10°C. An exothermic reaction took place with formation of a white precipitate. The mixture was allowed to adopt a temperature of +10°C, water (20 ml) was added and the mixture thus formed was extracted with three 25 ml portions of pentane. The solvent was evaporated from the combined extract phases to give a residue containing the title compound of Example II in a yield of 15%. The content of this compound in the residue was 39%. Comparison with Example II shows that the two-step procedure of Example II affords the title compound of this example in a considerably higher yield.

Comparative Example C

Triphenylphosphine (22 mmol.) was added with stirring at a temperature of −10°C to a solution of carbon tetrachloride (20 mmol.) in tetrahydrofuran (25 ml). No reaction was observed. After increasing the temperature to +10°C over a period of 10 minutes and cooling to −10°C n-heptanal (10 mmol.) was added and the temperature of the mixture was increased to +10°C. No reaction was observed. After increasing the temperature to 20°C and keeping the temperature at this level for 45 minutes, there was still no reaction. Then, the temperature of the mixture was increased to 67°C (reflux temperature) and kept at this level for one hour. At the end of this period all of the n-heptanal and almost all of the triphenylphosphine had been converted, but no 1,1-dichloro-1-octene had been formed.

Thus, unlike tri(dimethylamino)phosphine, triphenylphosphine fails to give any 1,1-dichloro-1-octene (cf. Example II).

Comparative Experiment D

A solution of carbon tetrachloride (20 mmol.) and triphenylphosphine (22 mmol.) in tetrahydrofuran (15 ml) was kept at reflux temperature (67°C) for 2.25 hours. In this period a precipitate was formed and at the end of this period more than 90% of the triphenylphosphine had been converted. After cooling of the suspension thus obtained to −10°C n-heptanal (10 mmol.) was added, which caused the formation of a white precipitate. Eight minutes later, after the temperature had been allowed to rise to +13°C, the n-heptanal was fully converted. Diethyl ether (50 ml) was added to the reaction mixture, the precipitated triphenylphosphine oxide was filtered off and the solvent was evaporated from the filtrate to give a residue consisting of the title compound of Example II, triphenylphosphine oxide and triphenylphosphine. This residue was stirred with n-pentane (50 ml), the organic phase thus formed was washed with two 25-ml portions of water, the washed organic phase was dried over anhydrous magnesium sulphate and the solvent was evaporated from the dried organic phase to give a residue containing the title compound of Example II in a yield of 30%. The content of this title compound in the residue was 54%.

Comparison with Example II shows that replacement of triphenylphosphine by tri(dimethylamino)phosphine results in a considerably higher yield of the title compound of Example II.

Example III

*Preparation of 1,1-dichloro-1-octene using tri(diethylamino)phosphine*

Carbon tetrachloride (15.6 mmol.) was added with stirring at a temperature of −20°C to a solution of tri(diethylamino)phosphine (7.8 mmol.) in pentane (50 ml). This caused the formation of a white precipitate. The temperature of the suspension was allowed to rise to +5°C (10 min.). This ended the first step. Then, the suspension was cooled to −10°C and n-heptanal (3.9 mmol.) was added. This caused a slightly exothermic reaction and the temperature of the mixture was allowed to rise to +13°C. This ended the second step; the conversion of n-heptanal was 100%. Water (25 ml) was added with stirring and the organic phase was isolated, washed with 0.1 N aqueous hydrochloric acid (25 ml) and with three 25 ml portions of water and the washed organic layer was dried over anhydrous magnesium sulphate. The solvent was evaporated from the dried liquid giving a residue containing the title compound in a yield of 98%. The content of this compound in the residue was 58%.

Example IV

*Preparation of cis-ethyl 2-(2,2-dichlorovinyl)-3,3-dimethylcyclopropane carboxylate*

Tri(dimethylamino)phosphine (7.0 mmol.) was added dropwise over a period of 5 min. at 0°C to a solution of carbon tetrachloride (7.0 mmol.) in pentane (15 ml), which caused the formation of a white precipitate. This ended the first step. Then, ethyl cis-3-formyl-2,2-dimethylcyclopropanecarboxylate (3.2 mmol.) was added to the mixture over a period of three minutes, whilst the temperature was allowed to rise to 13°C. Stirring was continued for 10 minutes at 13°C. This ended the second step. The conversion of the starting ester was 90%. Water (25 ml) was added to the reaction mixture and the organic phase was isolated and dried over anhydrous sodium sulphate. The solvent was evaporated from the dried organic liquid, giving a residue fully consisting of the cis-configuration of the title ester (yield 90%).

Example V

*Preparation of 1,1-dichloro-1-octene using prepared tri(dimethylamino)phosphine*

(a) Preparation of tri(dimethylamino)phosphine

A solution of dimethylamine (2.22 mol.) in hexane (350 ml) was added with stirring over a period of 20 minutes to a solution of phosphorous trichloride (0.327 mol.) in hexane (200 ml) at a temperature of −20°C. This caused the formation of a white precipitate of dimethylamine hydrochloride and a temperature rise to +18°C. The mixture was stirred for 20 hours at 20°C, cooled to 0°C and the suspension was extracted with water (150 ml). The raffinate phase obtained was dried over anhydrous sodium sulphate, the sodium sulphate was filtered off and washed with hexane to give a solution (in total 580 ml) containing tri(dimethylamino)phosphine (yield 70%).

(b) Preparation of 1,1-dichloro-1-octene

Carbon tetrachloride (20 mmol.) was added at a temperature of −20°C to a solution (70 ml) of tri(dimethylamino)phosphine (23 mmol.) in hexane, which solution was prepared as described in (a). On increasing the temperature of the mixture to +10°C in 15 minutes' time, a white precipitate was formed. This ended the first step. After cooling of the suspension to −10°C heptanal (10 mmol.) was added, which caused an exothermic reaction. The mixture was allowed to adopt a temperature of 20°C, which took 30 minutes; the n-heptanal was quantitatively converted into 1,1-dichloro-1-octene. This completed the second step. Then, water (20 ml) was added and the organic layer was isolated, washed with 0.1 N aqueous hydrochloric acid (5 ml), and two 20-ml portions of water and dried over anhydrous magnesium sulphate. The solvent was evaporated from the dried liquid, giving a residue containing the title compound in a yield of 70%. The content of the title compound in the residue was 90%.

**Claims**

1. A process for the preparation of a di-(halo)-vinyl compound of the general formula:

$$R^1-\overset{\overset{H}{|}}{C}=CHal_2 \qquad (I)$$

wherein Hal represents a halogen atom and $R^1$ an alkyl group or a substituted cyclopropyl group of the general formula:

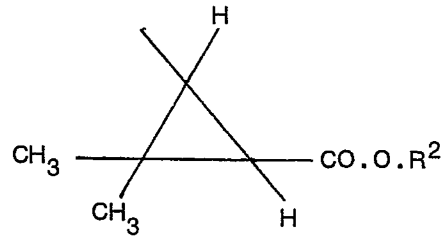

(II)

wherein $R^2$ represents a substituted or unsubstituted alkyl group, which process comprises reacting a tri(dialkylamino)phosphine with a tetrahalomethane and an aldehyde of the general formula:

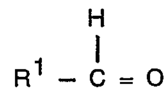

(III)

wherein $R^1$ has the same meaning as in the general formula I, in the presence of a substantially inert solvent, characterised in that (a) as a first step the tri(dialkylamino)phosphine is reacted with the tetrahalomethane in the absence of the aldehyde and (b) the product resulting from the first step is reacted with the aldehyde, steps (a) and (b) both being effected at temperatures in the range from −50°C to +50°C.

2. A process according to claim 1 further characterised in that steps (a) and (b) are both effected at temperatures in the range from −20°C to +35°C.

3. A process according to claim 1 or 2, further characterised in that the tri(dialkylamino)phosphine is tri(diethylamino)phosphine or tri(dimethylamino)phosphine.

4. A process according to any one of the preceding claims, further characterised in that the tri(dialkylamino)phosphine is prepared by reacting a dialkylamine with a phosphorous trihalide in the presence of a substantially inert solvent.

5. A process according to any one of the preceding claims, further characterised in that the substantially inert solvent is an alkane or tetrahydrofuran.

6. A process according to any one of the preceding claims, further characterised in that $R^1$ is an alkyl group containing less than 25 carbon atoms or a substituted cyclopropyl group of formula II, in which $R^2$ is an alkyl group containing less than 6 carbon atoms.

7. A process according to any one of the preceding claims, further characterised in that the aldehyde of formula III is *n*-hexanal, *n*-heptanal, *n*-decanal or ethyl 3-formyl-2,2-dimethylcyclopropanecarboxylate.

8. A process according to claim 6, further characterised in that the aldehyde of formula III is an alkyl (1R, cis)-3-formyl-2,2-dimethylcyclopropanecarboxylate.

**Revendications**

1. Procédé pour la préparation d'un composé de di-(halo)-vinyle de la formule générale:

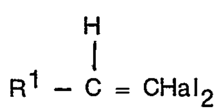

(I)

dans laquelle Hal représente un atome d'halogène et $R^1$ un groupe alcoyle ou un groupe cyclopropyle substitué de la formule générale:

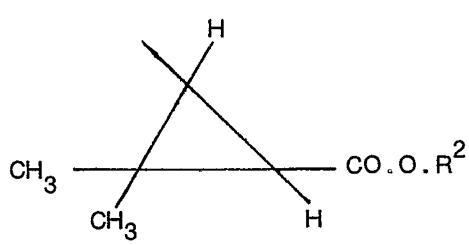

(II)

dans laquelle $R^2$ représente un groupe alcoyle substitué ou non, selon lequel on fait réagir une tri(dialcoylamino)phosphine avec un tétrahalométhane et un aldéhyde de la formule générale:

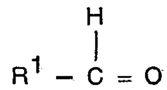

(III)

dans laquelle $R^1$ a la même signification que dans la formule générale I, en présence d'un solvant sensiblement inerte, caractérisé en ce que (a) comme première étape on fait réagir la tri(dialcoylamino)phosphine avec le tétrahalométhane en l'absence de l'aldéhyde et (b) on fait réagir le produit de la première étape avec l'aldéhyde, les étapes (a) et (b) étant effectuées toutes deux à des températures comprises entre −50°C et +50°C.

2. Procédé selon la revendication 1, caractérisé en ce que les étapes (a) et (b) sont effecuées toutes deux à des températures comprises entre −20°C et +35°C.

3. Procédé selon l'une des revendications 1 et 2, caractérisé en ce que la tri(dialcoylamino)phosphine est la tri(diéthylamino)phosphine ou la tri(diméthylamino)phosphine.

4. Procédé selon l'une des revendications précédentes, caractérisé en ce qu'on prépare la tri(dialcoylamino)phosphine en faisant réagir une dialcoylamine avec un trihalogénure de phosphore en présence d'un solvant sensiblement inerte.

5. Procédé selon l'une des revendications précédentes, caractérisé en ce que le solvant sensiblement inerte est un alcane ou le tétrahydrofuranne.

6. Procédé selon l'une des revendications précédentes, caractérisé en ce que $R^1$ est un groupe alcoyle contenant moins de 25 atomes de carbone ou est un groupe cyclopropyle substitué de formule II, dans lequel $R^2$ est un groupe alcoyle contenant moins de 6 atomes de carbone.

7. Procédé selon l'une des revendications précédentes, caractérisé en ce que l'aldéhyde de formule III est le *n*-hexanal, le *n*-heptanal, le *n*-décanal ou le 3-formyl-2,2-diméthylcyclopropane-carboxylate d'éthyle.

8. Procédé selon la revendication 6, caractérisé en ce que l'aldéhyde de formule III est un (1R, cis)-3-formyl-2,2-diméthylcyclopropanecarboxylate d'alcoyle.

**Patentansprüche**

1. Verfahren zur Herstellung von Di-(halogen)-vinyl-Verbindungen der allgemeinen Formel

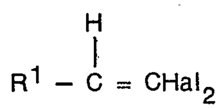

(I),

worin Hal für ein Halogenatom steht und $R^1$ eine Alkylgruppe oder eine substituierte Cyclopropylgruppe der allgemeinen Formel

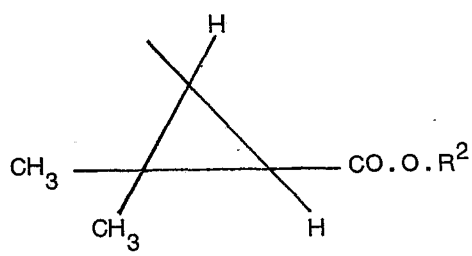

(II)

bedeutet, in der $R^2$ eine substituierte oder unsubstituierte Alkylgruppe ist, durch Umsetzung eines Tri-(dialkylamino)-phosphins mit einem Tetrahalogenmethan und einem Aldehyd der allgemeinen Formel

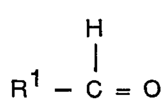

(III),

worin $R^1$ die gleiche Bedeutung wie in Formel I hat, in Anwesenheit eines im wesentlichen inerten Lösungsmittels, dadurch gekennzeichnet, dass man

(a) in einer ersten Stufe das Tri-(dialkylamino)-phosphin mit dem Tetrahalogenmethan in Abwesenheit des Aldehyds umsetzt und

(b) das in der ersten Stufe entstandene Produkt mit dem Aldehyd umsetzt, wobei die Stufen (a) und (b) bei Temperaturen im Bereich von −50 bis +50°C durchgeführt werden.

2. Verfahren nach Anspruch 1, ferner dadurch gekennzeichnet, dass die Stufen (a) und (b) beide bei einer Temperatur im Bereich von −20 bis +35°C durchgeführt werden.

3. Verfahren nach Anspruch 1 oder 2, ferner dadurch gekennzeichnet, dass das Tri-(dialkylamino)phosphin Tri-(diäthylamino)phosphin oder Tri-(dimethylamino)phosphin ist.

4. Verfahren nach einem der vorhergehenden Ansprüche, ferner dadurch gekennzeichnet, dass das Tri-(dialkylamino)phosphin hergestellt worden ist durch Umsetzung eines Dialkylamins mit einem Phosphortrihalogenid in Gegenwart eines im wesentlichen inerten Lösungsmittels.

5. Verfahren nach einem der vorhergehenden Ansprüche, ferner dadurch gekennzeichnet, dass das im wesentlichen inerte Lösungsmittel eine Alkan oder Tetrahydrofuran ist.

6. Verfahren nach einem der vorhergehenden Ansprüche, ferner dadurch gekennzeichnet, dass $R^1$ eine Alkylgruppe mit weniger als 25 Kohlenstoffatomen oder eine substituierte Cyclopropylgruppe der Formel II bedeutet, in der $R^2$ eine Alkylgruppe mit weniger als 6 Kohlenstoffatomen ist.

7. Verfahren nach einem der vorhergehenden Ansprüche, ferner dadurch gekennzeichnet, dass der Aldehyd der Formel III *n*-Hexanal, *n*-Heptanal, *n*-Decanal oder Äthyl-3-formyl-2,2-dimethylcyclopropan-carboxylat ist.

8. Verfahren nach Anspruch 6, ferner dadurch gekennzeichnet, dass der Aldehyd der Formel III ein Alkyl-(1R,cis)-3-formyl-2,2-dimethylcyclopropancarboxylat ist.